# EUROPÄISCHE PATENTSCHRIFT

(11) **EP 1 864 646 B1**
(45) Veröffentlichungstag und Bekanntmachung des Hinweises auf die Patenterteilung: **12.12.2012**
(21) Anmeldenummer: 07000688.7
(22) Anmeldetag: 15.01.2007
(51) Int. Cl.: A61K 8/44, A61K 8/04, A61Q 9/02, A61K 8/46, A61K 8/60, A61K 8/36, A61K 8/39

(54) **Rasierschaum im Pumpspender**
Shaving foam in a pump dispenser
Mousse à raser dans un distributeur à pompe

(30) Priorität: 13.01.2006 DE 102006001755
(43) Veröffentlichungstag der Anmeldung: 12.12.2007
(73) Patentinhaber: LOGOCOS Naturkosmetik AG, 31020 Salzhemmendorf (DE)
(72) Erfinder: Weiland-Groterjahn, Heinz-Jürgen, 31787 Hameln (DE)
(74) Vertreter: Lins, Martina

(56) Entgegenhaltungen:
- EP-A- 0 034 126
- WO-A-01/50910
- WO-A-2004/078359
- US-A1- 2004 161 447
- US-A1- 2005 112 084

## Beschreibung

Die Erfindung betrifft die Verwendung einer Mischung mit einem Tensidanteil für einen treibgasfrei in einem Schaum-Pumpspender (Foamer) aufschäumbaren Rasierschaum, auch in einem Pumpspender, der die aufzuschäumende, den Rasierschaum bildende Mischung enthält.

Seifenbasierte und tensidische Rasierhilfen werden in verschiedenen Darreichungsformen angeboten. Klassisch ist die Rasierseife, die mit einem Rasierpinsel aufgeschäumt wird. Da dies zeit- und kraftraubend ist, kamen mit der Entwicklung von Treibgasbehältern bald für Sprühschäume geeignete Formulierungen auf, wie z.B. in DT 2 422 937 offenbart. Die Sprühschäume sind sehr bequem in der Anwendung und können sehr feinblasig und stabil erhalten werden, was gelegentlich sogar zu dem Problem führt, dass diese Schäume für die Anwendung zu stabil sind, sich daher trocken und flockig anfühlen, die Haut nicht genügend benetzen und sich nicht gut verteilen lassen. Weiterhin sind Rasiercremes in der Tube bekannt, die jedoch vor der Anwendung mit Wasser und Rasierpinsel aufgeschäumt werden müssen.

Am Markt gewünscht sind Produkte, die einen klassischen Schaum bilden. Diese Schäume sollen sich angenehm und leicht anfühlen, hautpflegende Wirkung besitzen, die Barthaare für die Rasur vorbereiten, und nicht schwer auf der Haut kleben wie Gele oder Cremes. Der Schaum soll sich außerdem mit der Klinge leicht abziehen und leicht abwaschen lassen. Nach der Rasur soll ein angenehmes und gepflegtes Hautgefühl hinterbleiben.

Marktgängige Formulierungen in Cremeform oder als Druckgasformulierungen, die Seifen, bzw. verseifte Fettsäuren enthalten, sind immer alkalisch, was dazu führt, dass die Rasierklingen schneller stumpf werden und korrodieren. Auch ist die Hautverträglichkeit nicht optimal. Außerdem wird der hauteigene pH-Wert stark verändert und die Haut stark ausgetrocknet.

EP 0 034 126 A1 beschreibt eine Rasierhilfe, deren Konzept auf der Kombination einer kationischen organischen Ammoniumverbindung mit einem nichtionischen oder ampholytischen und dabei bevorzugt ethoxyliertem Tensid, Proteinen und Feuchthaltemitteln beruht.

Die WO 01/50910 A beschreibt ein Rasierverfahren und einen Dispenser, mit dem der Schaum direkt auf die Haut gesprüht wird, ohne mit der Hand verteilt werden zu müssen.

Die US 2004/161447 A1 betrifft eine Zusammensetzung für einen antibakteriellen/anitviralen Schaum in einem Foamer.

Die WO 2004/078359 A bezieht sich auf eine Dispenser, ohne dass konkrete Angaben zu Rasierschaumzusammensetzungen gemacht würden.

Die US 2005/112084 A1 beschreibt verschiedene topische kosmetische Zusammensetzungen, deren Gemeinsamkeit in der Anwesenheit verschiedener Vitamine zu sehen ist.

Die Aufgabe der Erfindung besteht darin, einen treibgasfrei aufschäumbaren Rasierschaum auf tensidischer Basis zur Verfügung zu stellen, der eine für das Rasieren gut geeignete Konsistenz besitzt, hautpflegend und umweltfreundlich ist. Diese Aufgabe wird durch die Verwendung einer Mischung für einen mit Hilfe eines Pumpspenders (Foamers) treibgasfrei abzugebenden Rasierschaum nach Anspruch 1 gelöst.

Das Produkt baut auf einer wässrigen Tensidlösung mit rein pflanzlichen Rückfettern auf, welche im pH-Wert beliebig eingestellt werden kann und sich vorzugsweise in einem hautfreundlichen, neutralen bis leicht sauren pH-Bereich befindet.

Die Tensidbasis besteht aus herkömmlichen und handelsüblichen anionischen oder nichtionischen Tensiden, jedoch unter Ausschluss von ethoxylierten Tensiden.

Vorzugsweise kann das Tensid oder wenigstens eines der eingesetzten Tenside ein Zuckertensid sein, oder eine Mischung aus Zuckertensiden. Als Tenside können beispielsweise Natriumlaurylsulfoacetat oder Natriumcocosulfat, Natrium- oder Ammoniumlaurylsulfat, Polyglycerylcaprate, Alkylpolyglucoside, Cocoglucosid (Kokosglucosid) sowie weitere in der Kosmetik bekannte Tenside eingesetzt werden. Es können auch Mischungen unter diesen oder mit anderen Tensiden Verwendung finden.

Für die Rückfettung werden in der zu einem Rasierschaum aufschäumbaren Mischung erfindungsgemäß Öle oder besondere, nicht hydrophile, d.h. in einer Emulsion mit Wasser zur Ölphase gehörige Rückfetter eingesetzt. Es werden keine Mineralöle sondern rein pflanzliche Öle wie Jojobaöl, Traubenkernöl, Mandelöl oder andere aus der Naturkosmetik bekannte Öle einzeln oder im Gemisch eingesetzt. Gut geeignete pflanzliche Rückfetter sind z. B. pflanzliche Fettsäuren, wie Myristinsäure, Laurinsäure, Stearinsäure oder Behensäure (nicht mit Alkalien verseift) beziehungsweise deren Ester oder Alkohole, allgemein C12-C28-Fettsäureester oder Fettsäurealkohole, auch Glyceryloleat und/oder Squalan.

Die erfindungsgemäße Mischung enthält weiterhin 4 bis 25 Gew.-% Ethanol und/oder ethanolisches Biomassedestillat, 4 bis 25 Gew.-% Polyole, ggf. Zusatzstoffe und ad 100 Gew.-% Wasser bezogen auf die aufschäumbare Mischung. Als ethanolisches Biomassedestillat kann bevorzugt Öko-Weizendestillat verwendet werden.

Vorzugsweise wird als Polyol oder im Gemisch mit anderen Polyolen Glycerin verwendet, weiter vorzugsweise in einer Menge von 10 bis 25 Gew.-%.

Das Ethanol bzw. die Bioalkohole, insbesondere ethanolische Biomassedestillat, dienen zur mikrobiologischen Stabilisierung und gleichzeitig als Lösungsmittelergänzung.

In bevorzugter Ausführungsform wird der Mischung zusätzlich wenigstens ein Verdickungsmittel zugesetzt. Als Verdickungsmittel sind natürliche Verdickungsmittel bevorzugt, insbesondere Xanthan, Carraghenan, Galactoarabinan, u.a. Verdickungsmittel oder Viskositätsgeber. Das Verdickungsmittel bewirkt in Kombination mit den Tensiden, der Öl- und der Wasserphase die Ausbildung einer Emulsion, die bei Abgabe aus dem Pumpspender aufgeschäumt wird und bei Einhalten der erfindungsgemäßen Rezeptur zu einem als Rasierschaum sehr zufriedenstellenden Schaumprodukt führt. Außerdem stabilisieren die Verdickungsmittel zusammen mit den Tensiden die Emulsion aus der erfindungsgemäßen Mischung, die sich zwischen polarer Phase (Wasser/Glycerin/Ethanol jeweils soweit vorhanden) und unpolarer Phase (Ölphase) in dem Pumpspender ausbildet.

Als Zusatzstoffe können sich vorzugsweise einer oder mehrere der folgenden Stoffe in der Mischung befinden:
Pflanzliche Extrakte, insbesondere als Hautpflegezusätze, wie z. B. Aloe Vera, Hamamelis, Ringelblume; ätherische Öle bzw. Duft- oder Riechstoffe als Wirkstoffe zur Parfümierung ;
Lösungsvermittler, insbesondere Polyglycerin-10-laurat;

Feuchtigkeitsspender, nämlich Harnstoff und Aminosäuren des "Natural Moisturizing Factor (NMF), Bindemittel, Antioxidantien; pH-Regulatoren, wie Citronensäure oder Citrate, z.B. Trinatriumcitrat, Milchsäure oder Lactate, z.B. Natriumlactat, von denen Einzelne oder mehrere verschiedene in unterschiedlichen Kombinationen in der Mischung eingesetzt werden können.

Vorzugsweise beträgt das Verhältnis von Tensid zu Polyol zwischen 0,2 und 2, weiter vorzugsweise zwischen 0,5 und 1,5. Das Verhältnis von Alkohol zu Polyol beträgt ebenfalls vorzugsweise zwischen 0,2 und 2,0, weiter vorzugsweise zwischen 0,5 und 1,5, weiter vorzugsweise um ca. 1. Die Mischung wird mit Wasser aufgefüllt, wobei die Inhalts- und Zusatzstoffe teilweise in wässriger Lösung zugemischt werden können. Das Wasser bildet mit den hydrophilen Inhalts- und Zusatzstoffen eine wässrige Phase, die mit den Ölen und rein fettlöslichen Bestandteilen der Mischung eine Emulsion bilden kann. Diese Emulsion wird durch die Tensid-Komponente mit Hilfe eines für das Aufschäumen geeigneten Pumpspenders aufgeschäumt. Gegebenenfalls wird die Mischung in dem Pumpspender vorher geschüttelt, um eine Emulsion zu bilden, die dann bei Abgabe durch den Pumpspender aufgeschäumt wird.

Die erfindungsgemäße Rasierschaum-Mischung entspricht den strengen Naturkosmetikkriterien des BDIH (Bundesverband der deutschen Industrie- und Handelsunternehmen).

Der erfindungsgemäße Rasierschaum hat folgende Vorteile:
- er entspricht den Standards für Naturkosmetik und ist biologisch gut abbau bar,
- er ist in einem Pumpspender (Foamer) optimal aufschäumbar und bildet einen stabilen, nicht zu leicht zerfließenden aber dennoch feuchten und gut hautbenetzenden Schaum,
- er ist gut verteilbar und fühlt sich angenehm an,
- er schont die Rasierklingen (wirkt nicht feststellbar korrosiv),
- er ist im gewünschten, i.a. neutralen bis leicht sauren pH-Bereich einstellbar und somit hautfreundlich,
- er bereitet die Barthaare gut für die Rasur vor,
- er ist rückfettend und dennoch gut abwaschbar.

Der Rasierschaum aus der erfindungsgemäß verwendeten Mischung liegt vorzugsweise in einem Pumpspender mit Reservoir vor. Das Reservoir sollte so dimensioniert sein, dass ein freies Volumen zum Aufschütteln vorhanden ist.

Bei dem Pumpspender an sich kann es sich um einen handelsüblichen Pumpspender für die Abgabe von Schäumen handeln (Foamer). Derartige Pumpspender bestehen im Allgemeinen aus einer Pumpspenderflasche, bzw. einem beliebig geformten Pumpspender-Behälter, der oder die mit einem schaumbildenden Abgabekopf versehen ist. Vom Abgabekopf ausgehend reicht ein Steigrohr in die Flasche oder den Behälter hinein, so dass dieses Rohr möglichst in jedem Füllungszustand in die aufzuschäumende Flüssigkeit oder Emulsion eintaucht. Der Abgabekopf umfasst weiterhin wenigstens ein Ventil mit Abgabedüse und eine Druckaufgabeeinrichtung, mit der von Hand durch im Allgemeinen mehrfaches betätigen der Einrichtung Druck auf den Behälter gegeben wird, um den Inhalt in Portionen heraus zu treiben und dabei aufzuschäumen. Bei den meisten Pumpspendern ist vorheriges Schütteln erforderlich, was jedoch von der Art der Schaumdüse abhängig ist.

Solche Foamer sind im Handel erhältlich. Beispiele für schaumabgebende Pumpspender finden sich beispielsweise in der US 6,053,364 oder der EP 613728.

Die erfindungsgemäße Rasierschaum-Formulierung ist für die Verwendung in treibgasfrei betriebenen Pumpspender optimiert und führt zu Schäumen, die die an Rasierschäume zu stellenden Bedingungen gut erfüllen.

Im Folgenden wird die Erfindung anhand einiger Beispiele näher illustriert, die jedoch lediglich dem besseren praktischen Verständnis der Erfindung und nicht etwa einer Beschränkung der Erfindung auf diese Beispiele dienen sollen.

### BEISPIELE

Es werden tabellarisch Rezepturen für Mischungen angegeben, die in geeigneten, handelsüblichen Pumpspendem (Foamern) zu Rasierschäumen aufschäumbar sind.

### Beispiel 1

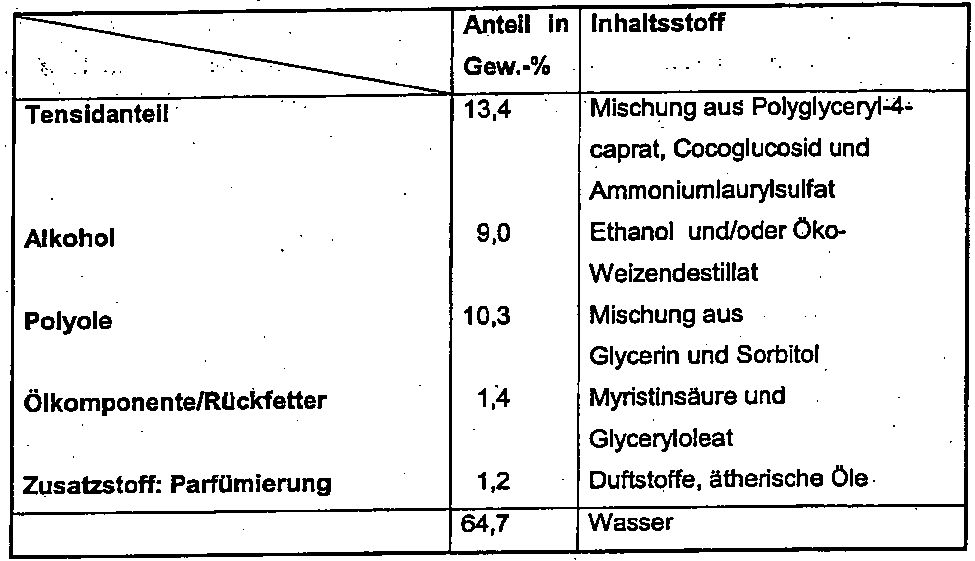

### Beispiel 2

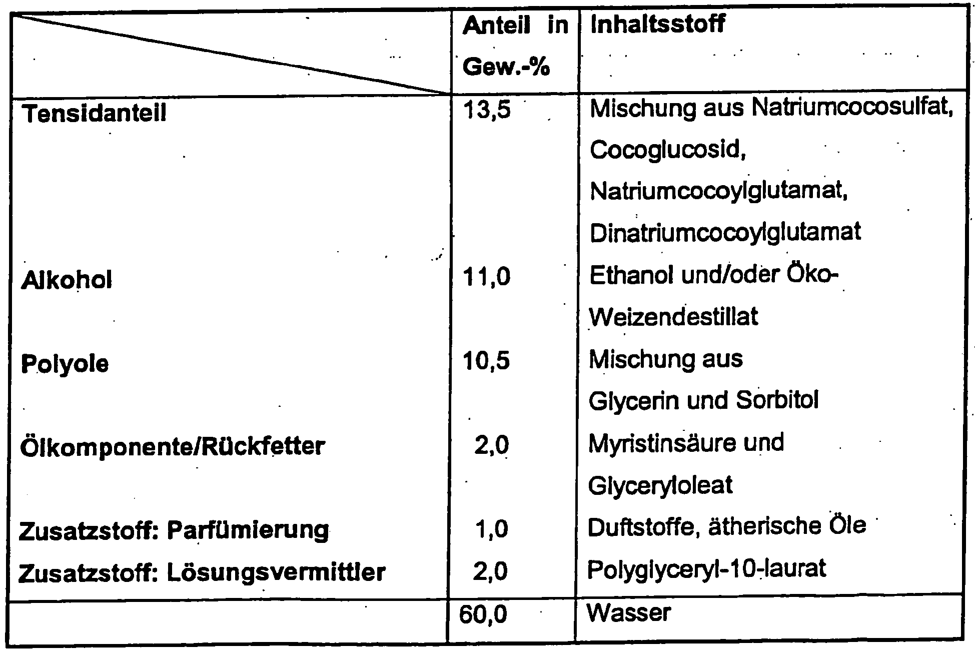

### Beispiel 3

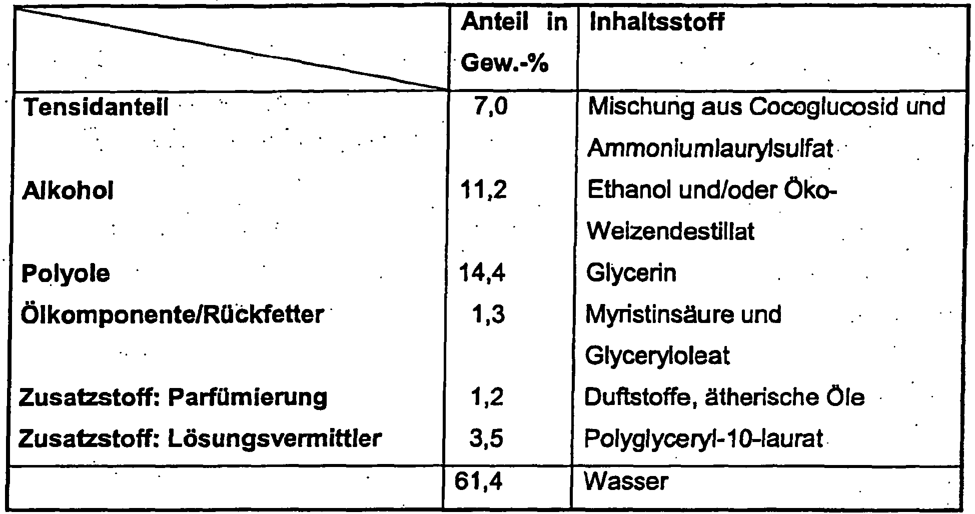

### Beispiel 4

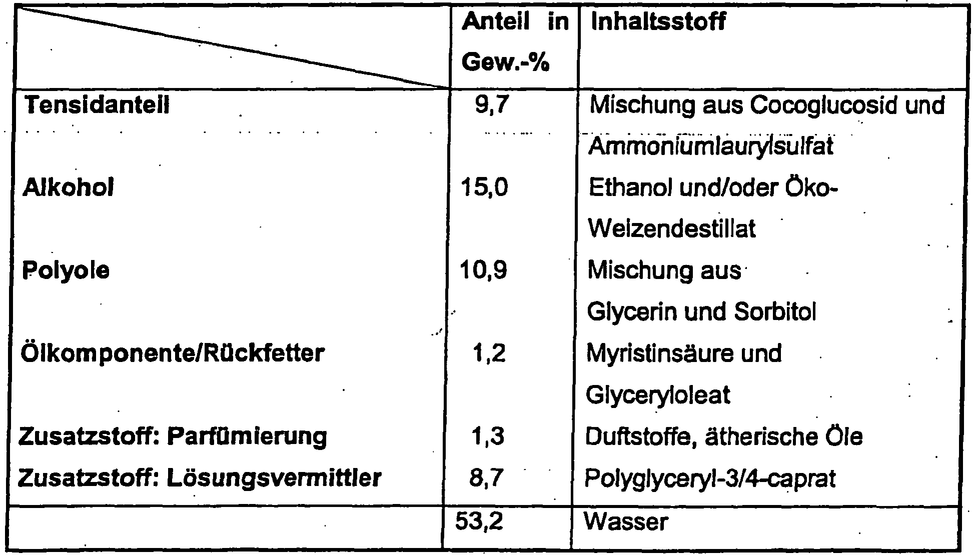

### Beispiel 5

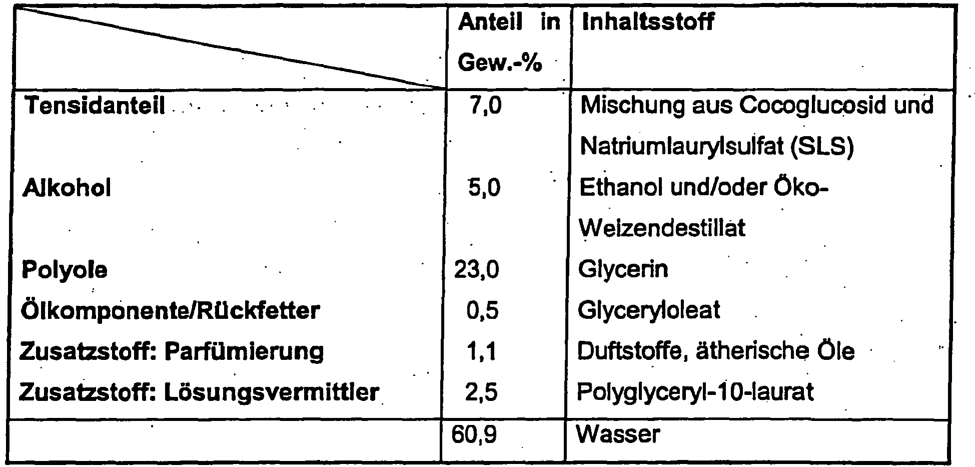

## Patentansprüche

1. Verwendung einer Mischung bestehend aus folgender Zusammensetzung:
| | |
|---|---|
| 5-25 Gew.-% | Tensidanteil aus wenigstens einem anionischen oder nichtionischen Tensid, oder einer Mischung aus beiden, unter Ausschluss von ethoxylierten Tensiden; |
| 0,5 - 15 Gew.-% | wenigstens eines Öls oder wenigstens eines kosmetischen Rückfetters, wobei die Rückfetter ausgewählt werden aus der Gruppe: pflanzliche Fettsäuren, deren Ester oder Alkohole, einschl. Glyceryloleat und Squalan, und wobei die Öle ausschließlich aus natürlich gewonnenen pflanzlichen Ölen bestehen, unter Ausschluss von Mineralölen und mineralölbasierten Rückfettern; |
| 4 bis 25 Gew.-% | Ethanol und/oder ethanolisches Biomassedestillat; |
| 4 bis 25 Gew.-% | Polyole, ausgewählt aus der Gruppe bestehend aus Glykolen, Triolen und Sorbitol, jeweils einzeln oder im Gemisch |
| je 0 - 10 Gew.-% | weitere Zusatzstoffe, ausgewählt aus der Gruppe bestehend aus: Feuchtigkeitsspendern, nämlich Harnstoff und Aminosäuren des "Natural Moisturizing Factor (NMF)"; Lösungsvermittlern; Bindemitteln; Antioxidantien; pflegenden Pflanzenextrakten; pH-Regulatoren und/oder Duftstoffen, von denen Einzelne oder mehrere verschiedene in der Mischung eingesetzt werden können; |
| ad 100 % | Wasser |
für einen mit Hilfe eines Pumpspenders treibgasfrei aufzuschäumenden und abzugebenden Rasierschaum.

2. Verwendung nach Anspruch 1, **dadurch gekennzeichnet, dass** der Gehalt an Zusatzstoffen 0 bis 5 Gew.-% beträgt.

3. Verwendung nach Anspruch 1 oder 2, **dadurch gekennzeichnet, dass** das Tensid oder wenigstens eines der Tenside ein Zuckertensid ist.

4. Verwendung nach einem der Ansprüche 1 bis 3, **dadurch gekennzeichnet, dass** als Polyol oder im Gemisch mit anderen Polyolen Glycerin verwendet wird, vorzugsweise in einer Menge von 10 bis 25 Gew.-%.

5. Verwendung nach einem der Ansprüche 1 bis 3, **dadurch gekennzeichnet, dass** als alkoholisches Biomassedestillat Öko-Weizendestillat verwendet wird, vorzugsweise allein oder in Kombination mit Ethanol in einer Menge von ca. 7 bis 20 Gew.-% bezogen auf die Rasierschaumzusammensetzung.

6. Verwendung nach einem der Ansprüche 1 bis 5, **dadurch gekennzeichnet, dass** der kosmetische Rückfetter der Ölkomponente, Myiristinsäure oder wenigstens einen C12-C20-Fettsäureester oder Fettsäurealkohol, Glycerololeat und/oder Squalan enthält.

7. Verwendung einer treibgasfrei aufschäumbaren Mischung gemäß der Ansprüche 1 bis 6 in einem Pumpspender mit Reservoir für die Mischung.

8. Verwendung nach Anspruch 7, **dadurch gekennzeichnet, dass** das Reservoir des Pumpspenders so dimensioniert ist, dass eine freies Volumen zum Aufschütteln der aufschäumbaren Mischung vorhanden ist.

## Claims

1. Use of a mixture consisting of the following composition:
5-25% by weight of surfactant component of at least one anionic or nonionic surfactant or of a mixture of both, with the exclusion of ethoxylated surfactants;
0.5-15% by weight of at least one oil or at least one cosmetic superfatting agent, where the superfatting agents are selected from the group: vegetable fatty acids, their esters or alcohols, including glyceryl oleate and squalane, and where the oils consist exclusively of naturally obtained vegetable oils, with the exclusion of mineral oils and mineral-oil-based superfatting;
4 to 25% by weight of ethanol and/or ethanolic biomass distillate;
4 to 25% by weight of polyols, selected from the group consisting of glycols, triols and sorbitol, in each case individually or as a mixture,
in each case 0-10% by weight of further additives, selected from the group consisting of: moisturizers, which are urea and amino acids of the "natural moisturizing factor (NMF)"; solubilizers; binders; antioxidants; caring plant extracts; pH regulators and/or fragrances, of which individual or a plurality of different ones can be employed in the mixture;
to 100% water for a shaving foam which can be foamed and dispensed with the aid of a pump dispenser, without the use of propellants.

2. Use according to Claim 1, **characterized in that** the additive content is 0 to 5% by weight.

3. Use according to Claim 1 or 2, **characterized in that** the surfactant or at least one of the surfactants is a sugar surfactant.

4. Use according to any of Claims 1 to 3, **characterized in that** glycerol is used as polyol or in admixture with other polyols, preferably in an amount of from 10 to 25% by weight.

5. Use according to any of Claims 1 to 3, **characterized in that** the alcoholic biomass distillate is eco-wheat distillate, preferably alone or in combination with ethanol in an amount of approximately 7 to 20% by weight, based on the shaving foam composition.

6. Use according to any of Claims 1 to 5, **characterized in that** the cosmetic superfatting agent of the oil component comprises myristic acid or at least one C12-C20-fatty acid ester or fatty acid alcohol, glycerol oleate and/or squalane.

7. Use of a mixture which can be foamed without the use of propellants, according to Claims 1 to 6, in a pump dispenser with a reservoir for the mixture.

8. Use according to Claim 7, **characterized in that** the dimensions of the reservoir of the pump dispenser are such that a free volume is available for shaking up the foamable mixture.

## Revendications

1. Utilisation d'un mélange consistant en la composition suivante :
| | |
|---|---|
| 5 - 25 % en poids | d'une fraction tensioactive à base d'au moins un tensioactif anionique ou non ionique, ou d'un mélange des deux, à l'exclusion de tensioactifs éthoxylés ; |
| 0,5 - 15 % en poids | d'au moins une huile ou d'au moins un agent de regraissage cosmétique, l'agent de regraissage étant choisi dans le groupe : acides gras végétaux, leurs esters ou alcools, y compris l'oléate de glycéryle et le squalane, et les huiles consistant exclusivement en huiles végétales obtenues de façon naturelle, à l'exclusion d'huiles minérales et d'agents de regraissage à base d'huiles minérales ; |
| 4 à 25 % en poids | d'éthanol et/ou de distillat éthanolique de biomasse ; |
| 4 à 25 % en poids | de polyols, choisis dans le groupe constitué par des glycols, des triols et le |
| | sorbitol, chacun individuellement ou en mélange |
| 0 - 10 % en poids chacun : | d'autres additifs, choisis dans le groupe constitué par : des hydratants, à savoir urée et acides aminés du « Natural Moisturizing Factor (NMF) » ; des tiers solvants ; des liants ; des antioxydants ; des extraits végétaux de soin ; des régulateurs du pH et/ou des parfums, dont les substances individuelles ou plusieurs substances différentes peuvent être utilisées dans le mélange ; |
complément à 100 % avec de l'eau
pour une mousse de rasage destinée à être libérée et transformée en mousse sans gaz propulseur à l'aide d'un distributeur à pompe.

2. Utilisation selon la revendication 1, **caractérisée en ce que** la teneur en additifs vaut de 0 à 5 % en poids.

3. Utilisation selon la revendication 1 ou 2, **caractérisée en ce que** le tensioactif ou au moins l'un des tensioactifs est un tensioactif glucidique.

4. Utilisation selon l'une quelconque des revendications 1 à 3, **caractérisée en ce qu'**on utilise comme polyol ou en mélange avec d'autres polyols du glycérol, de préférence en une quantité de 10 à 25 % en poids.

5. Utilisation selon l'une quelconque des revendications 1 à 3, **caractérisée en ce qu'**on utilise comme distillat alcoolique de biomasse du distillat de blé écologique, de préférence seul ou en association avec de l'éthanol en une quantité d'environ 7 à 20 % en poids, par rapport à la composition de mousse de rasage.

6. Utilisation selon l'une quelconque des revendications 1 à 5, **caractérisée en ce que** l'agent de regraissage cosmétique du composant huileux contient de l'acide myristique ou au moins un ester d'acide gras en C₁₂-C₂₀ ou alcool dérivé d'acide gras, de l'oléate de glycérol et/ou du squalane.

7. Utilisation d'un mélange transformable en mousse sans gaz propulseur, selon les revendications 1 à 6, dans un distributeur à pompe avec réservoir pour le mélange.

8. Utilisation selon la revendication 7, **caractérisée en ce que** le réservoir du distributeur à pompe est dimensionné de telle sorte qu'un volume libre est présent pour le secouement du mélange transformable en mousse.
